# EUROPEAN PATENT SPECIFICATION

(11) **EP 4 072 450 B1**
(45) Date of publication and mention of the grant of the patent: **07.02.2024**
(21) Application number: 20838604.5
(22) Date of filing: 09.12.2020
(51) Int. Cl.: A61B 17/70, A61B 17/00

(54) **AN ORTHOPEDIC SCREW EXTENDER HAVING A U-SHAPED BENT STRUCTURE WITH A REDUCED DIAMETER**
ORTHOPÄDISCHER SCHRAUBENSTRECKER, DER EINE U-FÖRMIGE GEBOGENE STRUKTUR MIT REDUZIERTEM DURCHMESSER AUFWEIST
PROLONGATEUR DE VIS ORTHOPÉDIQUE AYANT UNE STRUCTURE COURBÉE EN FORME D'U ET UN DIAMÈTRE RÉDUIT

(30) Priority: 11.12.2019 WO PCT/IB2019/060665
(43) Date of publication of application: 19.10.2022
(73) Proprietor: Neo Medical SA, 1096 Villette (CH)
(72) Inventor: BEYER, Morten, 8840 Rødkærsbro (DK)
(74) Representative: Weihs, Bruno Konrad
(86) International application number: PCT/IB2020/061698
(87) International publication number: WO 2021/116937

(56) References cited:
- US-A1- 2018 235 677
- US-A1- 2018 256 212
- US-A1- 2019 046 287
- US-A1- 2019 343 558

## Description

### CROSS-REFERENCE TO RELATED APPLICATIONS

The present invention claims priority to International Patent Application No. PCT/IB2019/060665 filed on December 11, 2019.

### FIELD OF THE INVENTION

The present invention relates to orthopedics and more precisely to orthopedic tools and systems including screw extenders to hold and operate with pedicle screws, rods and corresponding set screws. The invention also relates to instruments which are used for manipulating these elements, and methods of using these elements, to hold a pedicle screw to a screw extender, as well as methods of manufacturing the screw extender.

### BACKGROUND

In the field of orthopedics and implant tools and systems for orthopedic surgery, more specifically spinal fusion surgery for a spinal column, a pedicle screw is used to attach to a vertebrae with a bone anchor through an incision location on a back of the patient. After several pedicle screws are attached to different vertebrae, the heads of these pedicle screws are connected together via rod-type or bar-type device, and the rod-type or bar-type device, also called spinal rod, is attached to the head of the pedicle screws with a set screw. As an example, for several adjacent vertebrae for vertebrae fusion, for each vertebra a pedicle screw is screwably attached thereto with the bone anchor of the pedicle screw, and thereafter, these pedicle screws are mechanically fastened towards each other by the use of the spinal rod that is placed in a groove or U-shaped opening that is formed by the pedicle screw head, forming a row of pedicle screws along the spine. This allows to provide for the mechanical support needed for spinal stabilization for spinal fusion in a patient or living being.

To better reach into the incision location and screwably attach a pedicle screw to the vertebrae, the pedicle screw, specifically the head of the pedicle screw, is usually removably attached to a screw extender. The screw extender has the purpose to add additional length to the head of the pedicle screw allowing the operator or surgeon to act outside of the surgical incision, to keep the surgical incision open, but also to help guiding different tools and the spinal rod to the head of the pedicle screw. The screw extender that is configured to hold the pedicle screw is usually a tubular, longitudinal device that is quite a bit larger than the head of the pedicle screw, and itself has a longitudinally-shaped slot along a side thereof. When the pedicle screw head is connected to the screw extender, the longitudinally-shaped slot matches with the U-shaped opening in the screw head of the pedicle screw, and therefore allows to guide the spinal rod into the U-shaped opening through the longitudinally-shaped slot. The process of pushing down the spinal rod within the longitudinally shaped slot of the screw extender towards and into the head of pedicle screw is also called rod reduction.

For example, U.S. Patent No. 10,058,355 describes an orthopedic implant kit that provides for a pedicle screw, a corresponding set screw, a rod, and the tools to operate these, including a screw extender for holding the pedicle screw, and a set screw driver for threadably tightening the set screw relative to screw head of pedicle screw. U.S. Patent No. 7,160,300 describes a rod reduction method where intermediate guide tools are attached to bone screws, the intermediate guide tools having a tubular shape with a longitudinally-shaped channel that can guide a rod from the guide tools to the bone screw attached thereto. As another example, U.S. Patent No. 8,795,283 describes another type of kit orthopedic surgery system for surgical intervention for spinal stabilization, including pedicle screw with a head for receiving a rod, and tools necessary for the surgical intervention. The screw extender is made of a tube having two separable half-shells that are held together by a holding ring so that a tubular shape can be formed. In yet another example, U.S. Patent No. 8,262,662 provides for a system and method for delivering a spinal connector spinal anchor sites in a spinal column. In one embodiment, a spinal implant and access device is provided that includes a U-shaped receiver member, a bone-engaging member, an extension member, a spinal rod, and a set screw. The extension member has a tubular shape.

Similar orthopedic spinal surgery concepts, tools and devices have been proposed as discussed above, for attaching a rod to a pedicle screw via a set screw, for example U.S. Patent No. 5,129,388, U.S. Patent No. 5,520,689, U.S. Patent No. 5,536,268, U.S. Patent No. 5,720,751, U.S. Patent No. 5,984,923, U.S. Patent No. 6,056,753, U.S. Patent No. 6,183,472, U.S. Patent No. 6,258,090, U.S. Patent No. 6,454,768, U.S. Patent No. 6,648,888, U.S. Patent No. 6,740,086, U.S. Patent No. 7,618,442, U.S. Patent No. 8,308,782, U.S. Patent No. 8,876,868, U.S. Patent Publication No. 2006/0025771, and U.S. Patent Publication No. 2018/0289397.

A screw extender for an implant system with the features as defined in the preamble of claim 1 is known from US 2018/235677 A1.

However, the state of the art orthopedic tools, and the screw extenders, still present specific problems with respect to the manufacture, use, and operability of the screw extenders. For example, screw extenders can be bulky and relatively expensive to make, as they may be formed from a single tubular piece of machined metal or molded plastic, for example stainless steel or a titanium-based material. Also, given their tubular structure with side walls of a relatively large diameter, they require a lot of material and can be relatively stiff. Another disadvantage is the poor recyclability of these screw extenders, especially when employed in a surgery for one-time use. These and other reasons lead to a relatively inefficient and cost-ineffective manufacturing the screw extender. Therefore, despite all of the solutions currently proposed in the state of the art related spinal surgery tools and the corresponding screw extenders, strongly improved screw extenders, implant kits, and methods for manufacturing the screw extenders are desired.

### SUMMARY

The invention is defined in the independent claims 1 and 10 while preferred embodiments are set forth in the dependent claims.

According to the object of the present invention, a screw extender for an orthopedic implant kit is provided. Preferably, the screw extender includes two side walls facing each other, the two side walls connected to each other at the proximal side by a connection element, wherein inner sides of portions of the two side walls that face each other include a threading, and wherein the threading includes a plurality of flanks, each flank including a tab that is bent inwardly towards the other side wall and a wall-traversing opening in the corresponding side wall delineating the flank.

Also according to the object of the present invention, a method of manufacturing a screw extender for an orthopedic implant kit is provided. Preferably, the method includes the steps of cutting a strip of metal material to form two sets of wall-traversing openings, the sets of wall-traversing openings arranged to be axisymmetric to each other, the wall-traversing openings delineating tabs, axially bending the strip of material to have a curved cross-section with a bending radius, folding the strip of s material at a first location to form a 90° angle and at a second location to form another 90° angle to provide for a U-shaped structure having two parallelly-arranged walls, the sets of wall-traversing openings arranged to face each other, and inwardly bending the tabs to form flanks for an internal thread.

A method of manufacturing an internal thread into metal materia is also described herein but not claimed . The method preferably comprising the steps of cutting two plates of metal material to form two sets of wall-traversing openings, one set arranged in one of the two strips, the other set in the other one of the two strips, the wall-traversing openings delineating tabs, axially bending the strip of material around a longitudinal axis of extension of the two strip of material, to have a curved cross-section with a bending radius, and bending the tabs to the concave side of a cylindrical surface that is defined by the bent strip of material to form flanks of the internal thread.

The above and other features and advantages of the present invention and the manner of realizing them will become more apparent, and the invention itself will best be understood from a study of the following description with reference to the attached drawings showing some preferred embodiments of the invention.

### BRIEF DESCRIPTION OF THE SEVERAL VIEWS OF THE DRAWINGS

The accompanying drawings, which are incorporated herein and constitute part of this specification, illustrate the presently preferred embodiments of the invention, and together with the general description given above and the detailed description given below, serve to explain features of the invention.
FIG. 1A an exemplary top view of a strip or bar of material 90 that can be bend or shaped to form a screw extender 100, for example a stainless steel strip or bar, having different features formed therein, including threading structures, central hole, ridge or groove for holding a pedicle screw;
FIG. 1B shows a side view of an exemplary pedicle screw 5 having a screw head for attachment to the screw extender 100;
FIG. 1C exemplarily shows a side view of the U-shaped screw extender 100, made from the strip or bar of material 90, that is attached to the pedicle screw 5 with engagement mechanism for holding the pedicle screw 1, for example longitudinally-extending slots or grooves that engage with corresponding ridges, or longitudinally-extending ridges that engage with corresponding slots or grooves;
FIG. 1D shows an exemplary cross-sectional view of the screw extender 100 depicting two semi-circular or sections of cylindrical inner surfaces of the side walls 10, 20, and a fold at each end;

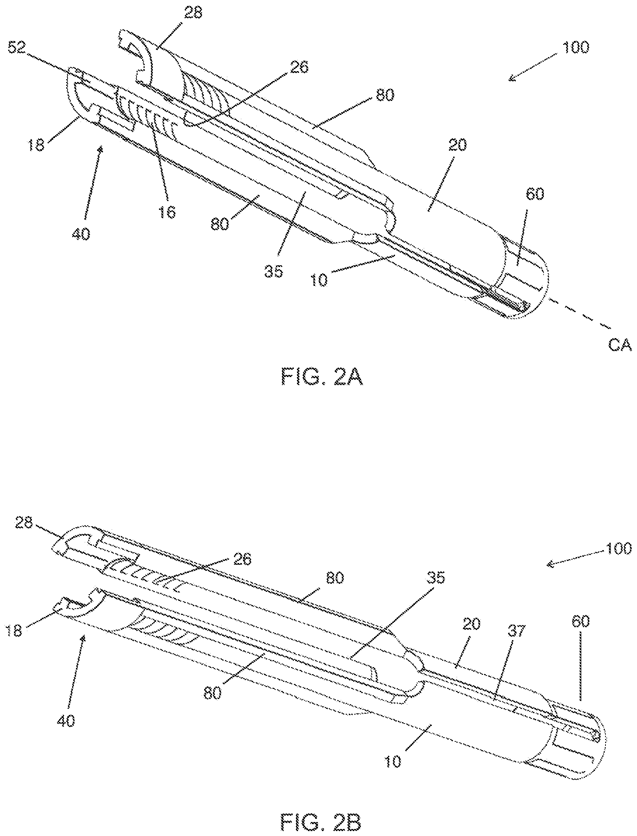

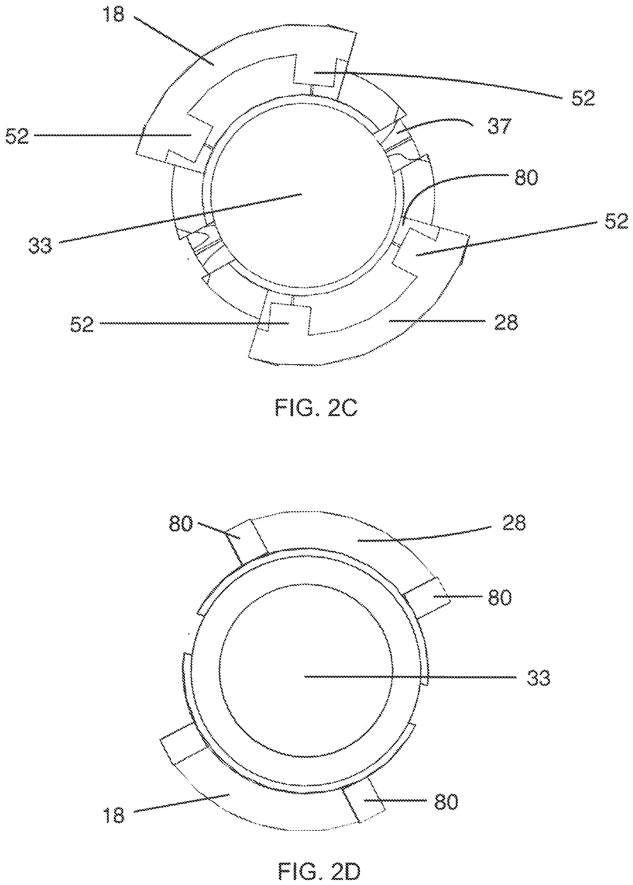

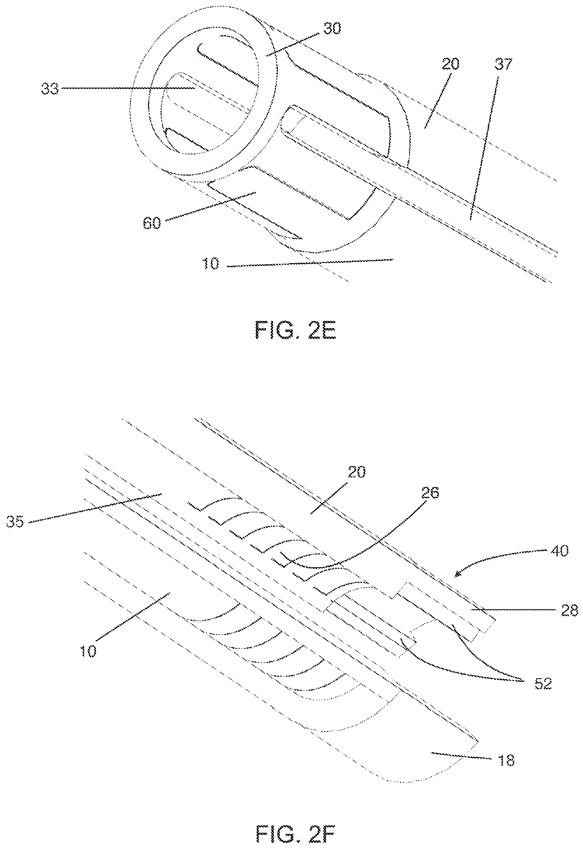

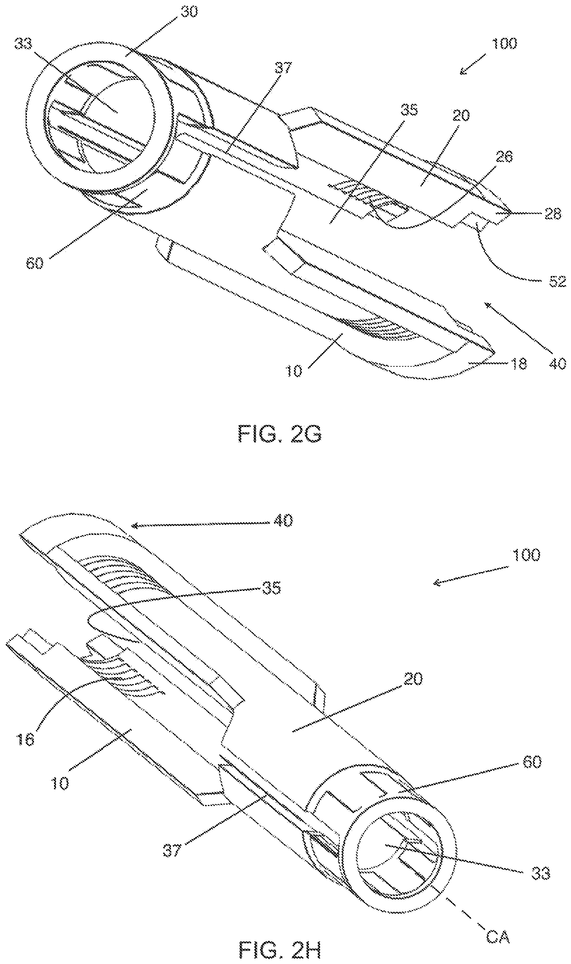

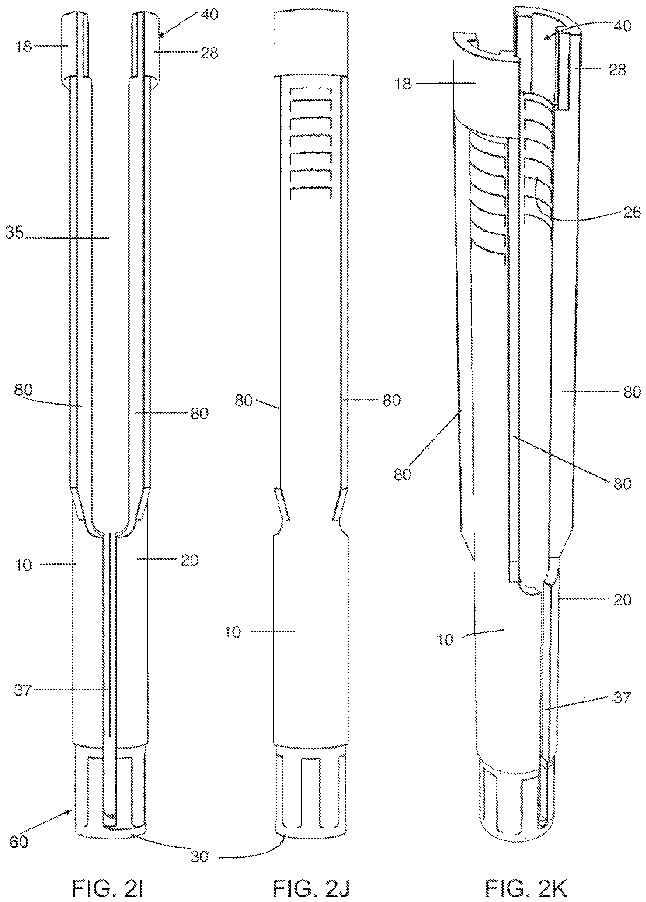

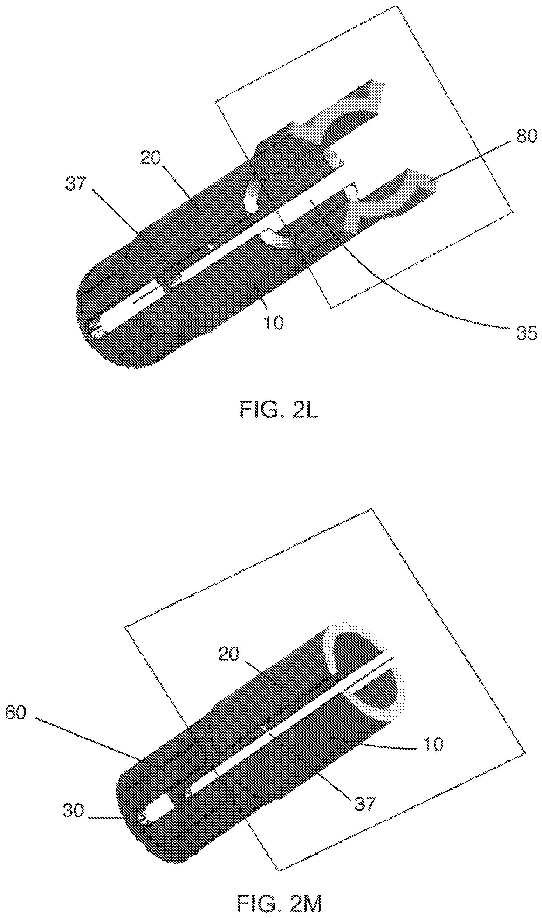

FIG. 2A to 2M depict another embodiment of the screw extender 100, showing different views, including front or proximal end perspective views (FIGs. 2A, 2B), back or distal end perspective views (FIGs. 2G, 2H), a front or proximal view showing engagement mechanism 40 (FIG. 2C), a back or distal view (FIG. 2D), partial perspective views of the back or distal end and front or proximal end, respectively (FIGs. 2E, 2F), different side views from different angles (FIGs. 2I, 2J, and 2K), and two cross-sectional cut-off views of the screw extender 100 (FIGs. 2L and 2M) to visualize two different cross-sections, by a cut plane that is perpendicular to an axis of longitudinal extension or main axis of the screw extender 100 at two different locations;
FIG. 3 shows a cross-sectional view of another embodiment of screw extender 100, where screw extender 100 has generally a square- or rectangularly-shaped cross-sectional shape, with folds 180 arranged in parallel to each other;
FIG. 4A-4D show different exemplary views of an embodiment of the flanks of the threading structures 16, 26, with FIG. 4A showing a top view of a side wall with threading structures 16, 26, FIG. 4B showing a side view of showing bent tabs, ridges or ledges 168 forming the threading flanks, FIG. 4C showing a cross-sectional side view along line CC showing bent tabs, ridges or ledges 168 forming the threading flanks and wall-traversing openings 164, and FIG. 4D showing a cross-sectional side view along line CC showing tabs 168 and openings 164 before the bending;

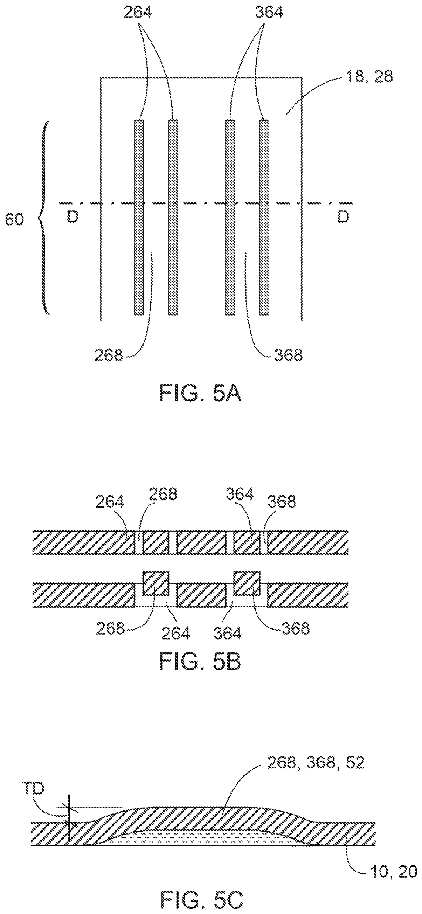

FIGs. 5A-5C showing different exemplary views of an embodiment depicting handle attachment mechanism 60 or screw head attachment mechanism 55, with FIG. 5A showing a top view of the proximal side of walls 10, 20 showing ridges 268, 368 (alternatively ridges 52 that form screw head attachment mechanism), and FIG. 5B showing a cross-sectional side view along line DD with the upper view showing ridges 268, 368 before being bent away from the plane formed by wall 10, 20, and FIG. 5B showing a cross-sectional side view along line DD with the upper view showing ridges 268, 368 after being bent, and FIG. 5C showing a cross-sectional view of a ridge 268, 368 along a cut line that is perpendicular to line DD formed by bending out the tab between the slits 264, 364;
FIGs. 6A and 6B shows bending dies 620, 630 for bending the ridges 268, 368 or the tabs 168 away from plane of extension of the walls 10, 20, with FIG. 6A showing a cross-sectional view that is perpendicular to an axis of longitudinal extension CA of the screw extender 100 being formed, and FIG. showing a cross-sectional view in a direction of the axis of longitudinal extension CA;
FIG. 7 shows another embodiment of the present invention, showing a proximal side of the screw extender 100 that is made of two separate parts by walls 10, 20, that can be interconnected with each other, by the use of two complementary cut sections or openings 464, 564 of the walls 10, 20; and
FIG. 8 shows a cross-sectional side view along axis CA of a screw extender 100 with a threaded device 105 located therein, for example a rod reduction tool, set screw driver, screw driver, or screw release instrument.

Herein, identical reference numerals are used, where possible, to designate identical elements that are common to the figures. Also, the images are simplified for illustration purposes and may not be depicted to scale.

### DETAILLED DESCRIPTION OF THE SEVERAL EMBODIMENTS

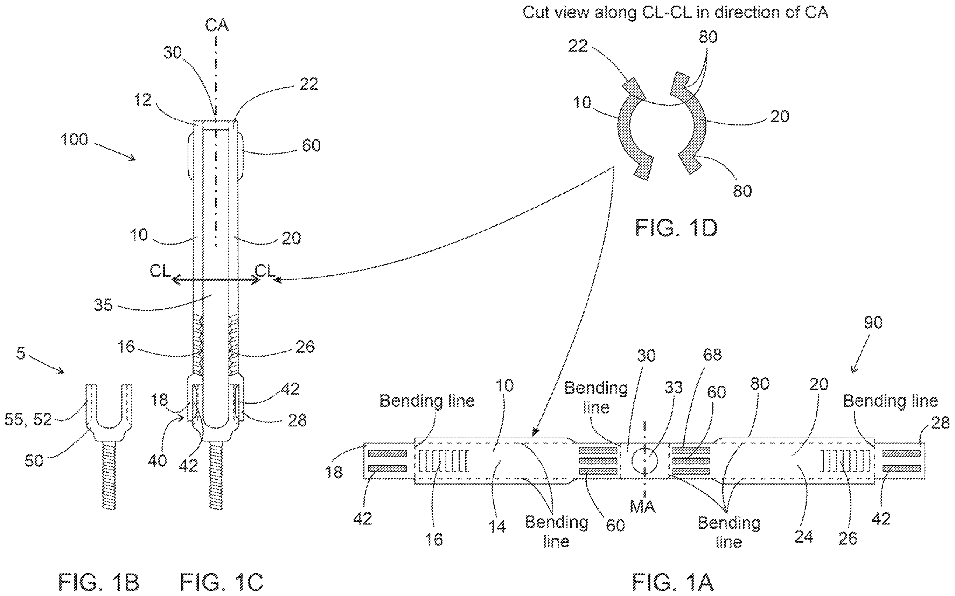

According to the present invention, a screw extender 100 for an orthopedic implant kit is provided, as exemplary shown in FIGs 1A, 1C, and 1D, configured to be removably attached to a pedicle screw 5. The screw extender 100 includes two side walls 10, 20 or legs facing each other, preferably arranged substantially parallel to each other. At a top or proximal end 12, 22 of the two side walls 10, 20, the two side walls 10, 20 are connected or fastened to each other with a central connection element 30. The top or proximal end is defined to be the end that will face the surgeon, operator, or user during the use of the screw extender 100 when operating with a pedicle screw 5. In this respect, the screw extender 100 forms or has a U-shape, with central connection element 30 forming a bottom of the U-shape. The U-shape provides for a longitudinal groove 35 along screw extender, for accommodating a spinal rod therein, for example a spinal rod that perpendicularly traverses screw extender 100 via longitudinal groove 35, and can also accommodate tools, such as set screw driver, rod reduction device, screw driver, torque-controlled driver within longitudinal groove 35 along the central axis CA, when inserted from a central hole or bore 33 arranged at central connection element 30. This is shown in U.S. Patent No. 10,058,355. The opening of the U-shape is configured to hold a screw head of a pedicle screw, as further explained below. The inner side surface 14, 24 of the two side walls 10, 20 include threading structures 16, 26, respectively, forming part of an internal thread.

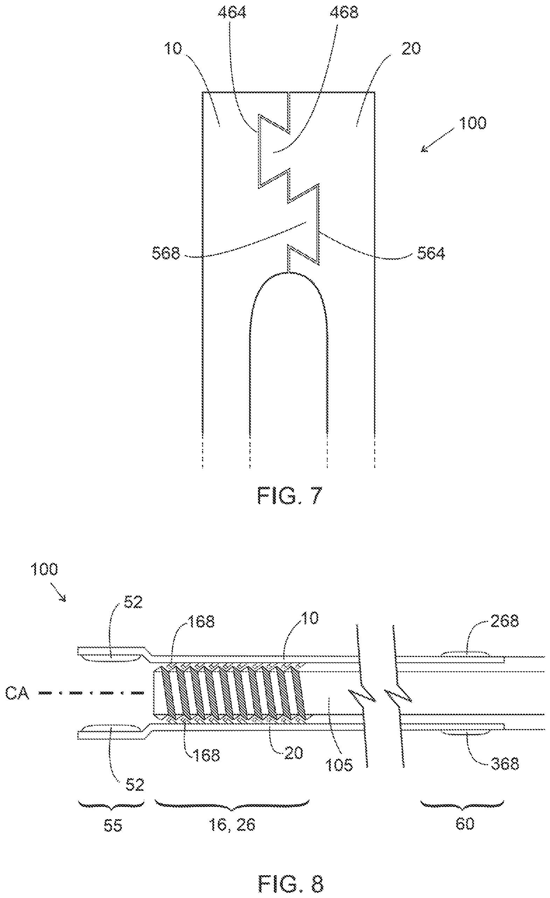

FIG. 1A shows the unfolded or flat strip or bar of material 90 that will form screw extender 100, with inner side surfaces 14, 24 and threading structures 16, 26 exposed. Bending lines are also indicated, being locations where the strip of material 90 can be bent or folded to form screw extender 100. Threading structures 16, 26 are formed such that they form an internal thread for a screw or threaded rod having a corresponding external thread to threadably engage with threading structures 16, 26, when the screw or threaded rod 105 engages inside the two side walls 10, 20, parallel to the longitudinal extension of the two side walls 10, 20, along central axis CA (FIG. 8). This requires that the side walls 10, 20 are arranged at a predefined distance to each other, defined by the length of central connection element 30, and are also arranged to be substantially parallel to each other. The side walls 10, 20 and central connection element 30 can be made of a single element, for example from a single strip or bar of material 90, for example a stainless steel material, having an exemplary thickness in a range of 1 mm - 3 mm, in some variants even less than 1mm. With respect to the overall diameter of the screw extender 100, the outer diameter can be less than 15mm, preferably in a range between 12 mm and 14 mm. Commonly used screw extenders for orthopedic surgeries have a diameter of 20 mm or more.

In the context of this description, threaded structures 16, 26 are described that are formed into two parallelly-arranged walls 10, 20 that form part of a screw extender 100 for orthopedic surgeries. However, the herein described structural arrangements and methods of manufacturing the threaded structures 16, 26 can be used for any other device that is made of two parallelly-arranged walls 10, 20 that face each other, and that are made of metal, for example but not limited to components for other types of surgical tools, automotive parts, machines, robotics, general tools, biking. For example, walls 10, 20 can be two separate elements, for example metal plates, blocks, or parts, that can be arranged in parallel to each other to form an internal threading with threaded structures 16, 26, held by additional parts. As another example, walls 10, 20, and central connection element 30 are three separate elements, that can be arranged to form a U-shape, with walls 10, 20 arranged parallel to each other, and are removably or irremovably interconnected to each other, either by an additional mechanical fastening or holding device, or are welded or soldered together, or attached to each other by other means, for example an adhesive.

Moreover, distal ends or portions 18, 28 of the two side walls 10, 20 include an engagement mechanism 40 for removably attaching a screw head 50 of a pedicle screw 5, via a complementary attachment mechanism 55, with complementary structural features to engagement mechanism 40, on screw head 50. Distal or bottom ends are defined as the ends of side walls 10, 20 that face the surgical incision, or the ends that face the vertebrae for attachment of pedicle screws 5. FIG. 1C shows an exemplary pedicle screw 5 attached to screw extender 100 via engagement mechanism 40 and attachment mechanism 55 of pedicle screw 5. In the variant shown, for example, the engagement mechanism 40 can be made of at least one groove or ridge 42, arranged at an inner surface of distal ends 18, 28 of the two side walls 10, 20, having an attachment mechanism 55 with complementary ridge or groove 52 arranged at outer side walls of screw head 50. This engagement mechanism 40, and thereby the distance between the first and the second side wall 10, 20 is configured such that it allows for a press-fit connection between screw 5 and screw extender 100, and screw 5 can be inserted by pressing the screw 5 towards the screw extender 100 along central axis CA for progressive insertion, and can be separated from screw extender 100 by pressing screw 5 downwards away from the distal ends of side wall 10, 20 for progressive separation.

Moreover, as exemplarily shown in FIGs. 1A and 1D, the single strip of material 90 that will form screw extender 100 can another structuration of the surfaces of side walls 10, 20 for removable attachment of a handle or other type of holding device, to form a handle attachment mechanism 60. For example, handle attachment mechanism 60 can include additional ridges or grooves 68 arranged in parallel with the central axis CA, at an area between the middle sections of the side walls 10, 20, and central connection element 30. The ridges or grooves 68 can be pressed or stamped into the material, or can be formed by machining away material from single strip of material 90, for example by a CNC machine or laser ablation, or other material removal processes. These ridges or grooves 68 serve to add an attachment mechanism for a handle, so that a handle can be placed perpendicularly to screw extender 100 allowing the user, operator, or surgeon to hold on to the screw extender 100, as shown exemplarily in U.S. Patent No. 10,058,355. The ridges or grooves 68 are shown in FIG. 1C at a top or proximal area of screw extender 100, close to the central connection element 30.

In a variant, to provide for a defined or controlled stiffness to screw extender 100, and also provide for the desired width of longitudinal groove 35, for example to be somewhat wider than a corresponding spinal rod that will be accommodated in groove 35 of screw extender 100, at middle sections of side walls 10, 20, the edges along a long side of side walls 10, 20 can be bent to form folds or flares 80. For example, bent folds or flares 80 can be forming by bending a portion along a long side of side walls 10, 20 along a bending line that is parallel to a longitudinal axis of extension CA of screw extender 100, with an additional bending step by a press. This is exemplarily shown in FIGs. 1A and 1D. These folds 80 allow to increase stiffness of screw extender 100, for example to reduce bending away from the central axis CA, in particular along the groove or opening 35, at the same time allowing to use a relatively thin material for strip 90, for example a material having a thickness of less than 3 mm, preferably around 1 mm, even below 1 mm. In addition, folds or flares 80 can provide for an enhanced grip of screw extender 100 when held by a user or operator. With respect to a bending angle of the flare 80 relative to a tangential plane at the cylindrical or curved wall 10, 20 of screw extender 100 at the bending line, as shown in FIG. 1D, the angle can be perpendicular or 90°, or in a range between 60° and 120°. Therefore, an overall volume of material that is used for screw extender 100 can also be reduced, using thinner wall material as compared to the classic tube-based solutions. In a variant, folds or flares 80 can be arranged to provide for an additional guiding structure for a spinal rod that will be located in the U-shaped longitudinal groove 35 formed by screw extender 100, spinal rod usually arranged to be perpendicular to the axis of longitudinal extension CA of screw extender 100, for example by arranging all four (4) flares to be parallel to each other, arranged substantially parallel to an axis of longitudinal extension of the spinal rod inside groove 35, thereby bending flares 80 away from by less than 90° from walls 10, 20, respectively.

The advantages of the herein described screw extender 100 that is made of a singular bar or strip of material 90 are manifold over the traditional tube-based screw extenders of the background art. For example, with such screw extender, it is possible to reduce the amount of material used to a minimum, and provides for a simple and cost-effective solution. In this respect, the screw extender 100 serves an important purpose for toolsets that are made for one-time use, and when disposed of can be recycled for making other products. It minimizes the materials necessary and thereby reduces the weight. Also, screw extender 100 herein described can be made narrower than the existing ones, using a strip of bent material to form the legs of walls 10, 20 of screw extender 100. Many background art screw extenders are made of a synthetic material, while this screw extender can be manufactured from a single piece of metal material, for example a trip of stainless steel, and can then be stamped, cut, machined, and bent to have its final U-shape with all the features.

According to the present invention, a method for manufacturing a screw extender 100 from a single piece of material is provided, for example a strip of metal 90. First, a metal sheet can be stamped or cut to form the general outline as shown in FIG. 1A. This can include the creation of central hole 33 Central hole 33 can have a diameter or radius that exceeds a diameter or radius of a major diameter or radius of an external thread of tool 1005. Next, the protruding or depressing features of strip of material 90 can be formed, including the ridges or grooves 60, engagement mechanism 40 with ridges or grooves 42, by passing the outline into a press, or by a removal or additive processing. Next or before the last step, the threading structures 16, 26 can be machined into strip of material 90. Folds 80 can be formed by bending or stamping. Thereafter, the screw extender 100 can be formed by bending it twice by 90° to form the U-shaped structure, at bending lines at the central connection portion 30.

FIGs. 2A to 2M show different views of another embodiment of screw extender 100, with screw extender 100 having an additional groove 37 that extends in the upper section of screw extender 100, and connects with the much wider groove or opening 35 that can accommodate the spinal rod (not shown). Along the entire groove 35, folds 80 are formed at each four edges of both of side walls 10, 20 for stiffening purposes. As can be seen in FIGs. 2C, 2D and 2L, the folds 80 are shown to protrude away from walls 10, 20 by a 90° degree angle, i.e. they are perpendicular to the curved portion of walls 10, 20, see also FIG. 1D. Moreover, distal ends or portions 18, 28 of the two side walls 10, 20 are shown to have arcuate sections that have a radius or diameter that is larger than the diameter of the walls 10, 20 at the groove 35, for accommodating the head of pedicle screw 5.

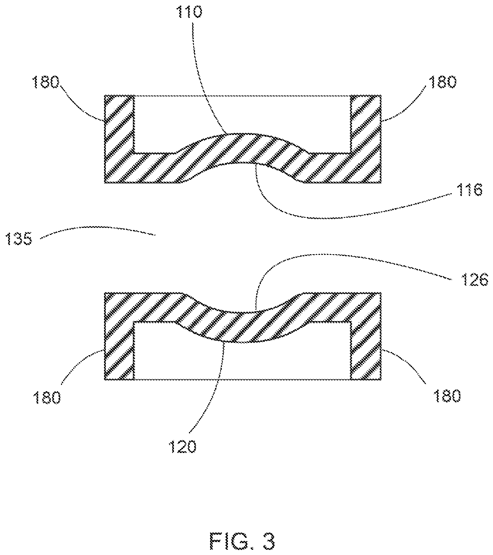

As illustrated by the cross-sectional views of FIGs. 2L and 2M, the walls of screw extender 100 formed by elements 10, 20 are relatively thin, and are shown to have an arcuate or curved shape, being a section of a cylinder. However, it is also possible that the walls have a square or rectangular cross-sectional shape, as illustrated in FIG. 3. In this variant, walls 110, 120 are bent or otherwise formed to have each two folds 180 that form the exterior part of side walls 110, 120, arranged perpendicularly to side walls 110, 120 when viewed in a direction of central axis CA. This also allows to provide for a channel, groove, or opening 135 that has side walls 110, 120 where at least a portion of those side walls 110, 120 are parallel to each other, extending in a direction of longitudinal extension of spinal rod that is arranged in groove 135, for better guidance of the spinal rod. Also, curved, or arcuate sections are still needed for accommodating a screw driver tool, and for having threading 116 and 126 that face each other, arranged centrally in walls 110, 120, when viewed in a direction of axis CA. The overall appearance of such screw extender can therefore be predominantly rectangular with a substantially square cross-section viewed in directions of axis CA, having an internal section cylindrical structure inside the groove 135 for guiding the screw driver or other tool, and for providing the threading 116, 126.

Moreover, in the variants shown in FIGs. 1 and 2, the two side walls 10, 20 are held together by central connection element 30, but it is also possible to have two separate elements for side walls 10, 20, that can be locked or otherwise interconnected together with a central connection element, for example a locking nut that engages with a thread at proximal portions 12, 22, a locking clamp, with an example of interlocking features of two elements shown in FIG. 7 as further discussed below.

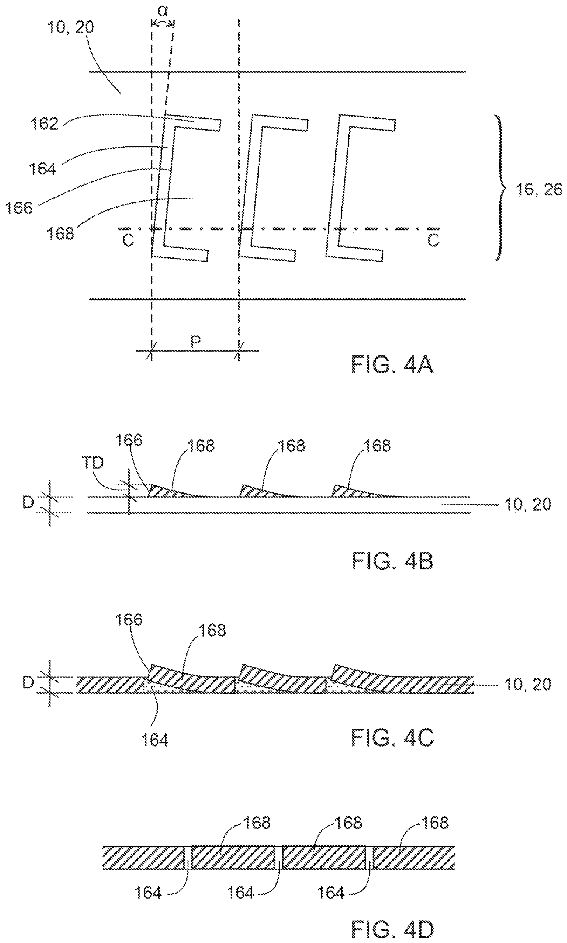

FIG. 4A-4D show different exemplary views of an embodiment of the flanks that form the threading structures 16, 26, exemplarily three (3) flanks of the threading structures 16, 26 are shown for one side wall 10 or 20, the number flanks being chosen to be low for visualization purposes. With the top view of FIG. 4A, it can be seen that wall-traversing openings 162, 164 are cut into the material that forms walls 10, 20, these openings 162, 164 delineating or defining a tab 168 in walls 10, 20. In the variant shown the wall-traversing openings 162, 164 form three slits or cuts that are arranged in a U-shape, to have a longer slit 164 and two shorter slits 162, so that a tab 168 is formed having an edge 166. The tab 168, in a bend state, forms the flanks of the threading structures 16, 26. A width of the slits 162, 164 can be less than 1mm. Longer slit 164 and edge 166 are formed such that they are arranged at an angle α relative to an axis that is perpendicular to axis CA, forming an oblique angle, having a length that can be between 5mm and 10 mm. Angle α corresponds to the helix angle of the thread for a corresponding instrument that will engage with threading structures 16, 26, and is preferably smaller than 15°. Moreover, the distance between the tabs 168 corresponds to a pitch P of the thread. Wall-traversing openings 162, 164 can be formed by different method steps, for example but not limited to laser ablation of the wall material, laser cutting of the wall material, by removal machining with a CNC machine or other type of metal processing removal technique, by stamping with a corresponding die and the press brake. After bending or pressing walls 10, 20 to a circular or arcuate cross-sectional shape, a press can be used to bend tabs 168 outwards away from wall 10, 20.

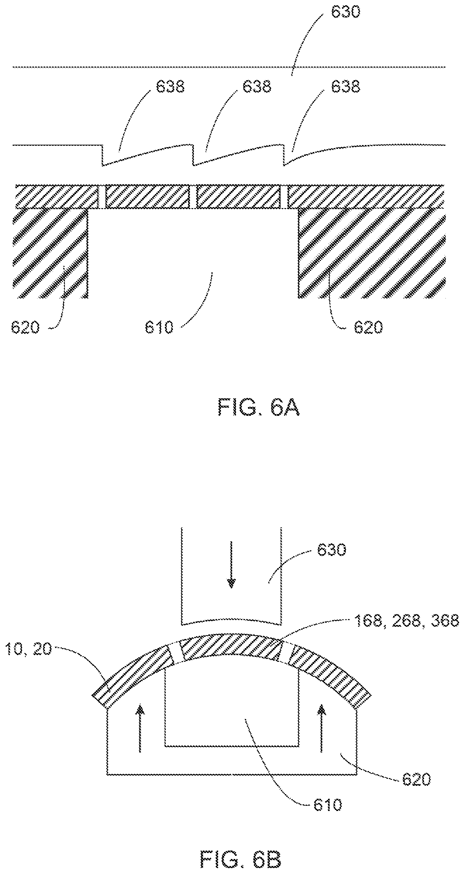

Next, with FIG. 4B a side view is shown of the bent tabs, ridges or ledges 168 that are forming the threading flanks of an internal thread, that are bent to slightly protrude away from a cylindrical surface or cylindrical plane that is formed by the wall 10, 20, to form a protrusion depth TD that is slightly smaller than a depth of a thread. The surface of edges 166 and tab 168 form a flank for the thread, that is integrated to the wall 10, 20 of the screw extender. With FIG. 4B, it seems that wall 10, 20 is flat, but preferably, wall 10, 20 has previously been bent to have a curved or arcuate shape in a cross-sectional view in direction of axis CA, as shown in FIG. 6B. FIG. 4C showing a cross-sectional side view along line CC showing bent tabs, ridges or ledges 168 forming the threading flanks and wall-traversing openings 164. Thickness D of wall 10, 20 an be chosen to be relatively thin, for example preferably more than 1mm and less than 3 mm, and is preferably made of metal material that can be used for surgeries, for example stainless steel. This allows to manufacture a screw extender 100 with an overall diameter that is strongly reduced as compared to the conventional screw extender made of plastic or other types of composite material. FIG. 4D showing a cross-sectional side view along line CC showing tabs 168 and openings 164 before the bending of the tabs 168 to form the flanks of threading structures 16, 26.

With the bent tabs 168 that form edges 166 for a threading structure 16, 26, as exemplarily visualized in FIG. 4C, it is possible to provide for an additional advantageous feature, compared to classic threadings. For example, a threaded device 105 that will engage with threading structures 16, 26 of screw extender 100 could be pushed or pulled against the slopes that are formed by the bent tabs 168 (in the visualization of FIG. 8 towards the left along axis CA) without any relative rotation, to bent them temporarily radially outwardly, requiring a certain threshold pulling or pushing force. This way threaded device 105 can be moved relative to screw extender 100 towards the left without the need of any threading action and rotation between threading structures 16, 26 of walls 10, 20, and threading of threaded device 105, as by the urging of flanks of the threading onto the tabs 168, tabs 168 will flex radially away from axis CA. In turn, if pushed or pulled to the other side (in the visualization of FIG. 8 towards the right along axis CA) because bent tabs 168 cannot flex backwards due to the abutment of the flanks of threaded device 105 against the edges 166, a threadable engagement and rotation between threaded device 105 and screw extender 100 is necessary, to further move threaded device 105 to the right, relative to screw extender 100. This allows to provide a ratchet effect between threading structures 16, 26 and the threaded device 105.

FIGs. 5A, 5B and 5C show different exemplary views of an embodiment the handle attachment mechanism 60 or the screw head attachment mechanism 55 that are formed by bending out portions of the wall 10, 20, with FIG. 5A showing a top view of the proximal side of walls 10, 20 showing ridges 268, 368 that are formed between by two parallel slits 264, 364 that form wall-traversing openings, the length of slits 264, 364 defining or delineating the ridges 268, 368. Ridges 52 that form screw head attachment mechanism 55 can be made analogously. In the variant shown, slits 264, 364 can have an exemplary length in a range between 5 mm and 20 mm, and can be formed analogously as discussed above with respect to slits 162, 164. After forming parallel slits 264, 364, walls 10, 20 can be bent to be have an arcuate or curved shape, and thereafter, the tabs between slits 264, 364 can be bent away from the plane or surface that forms walls 10, 20 to form ridges 268, 368, as shown in the cross-sectional view of FIG. 5B and 5C. For example, ridges 268, 368 are formed by bending the tabs or bars between slits 264, 364toutwardly in a radial direction away from a center axis CA, to form ridges 268, 368 that protrude away from outer surface of walls 10, 20. A protrusion height TD can be chosen based on the application, for example in a range between 0.5 mm to 3 mm.

FIGs. 6A and 6B shows bending dies 620, 630 for a press brake that allows to bend the ridges 268, 368 or the tabs 168 away from plane of extension of the walls 10, 20. Preferably, a bending die 630 on the pressing side has protruding bending teeth or protrusions 638 having a width that is somewhat smaller than a width of the ridges/tabs 168, 268, 368, and can also have a sloped saw-tooth like cross-section. In addition, the bending die on the holding side 620 can be formed to have an opening or cavity 610 that allows the ridges/tabs 168, 268, 368 to be bent to protrude inside the space formed by cavity 610. Moreover, the surfaces of bending dies 620, 630 have surfaces of contact that confirm with the curvature radius of the arcuate shape of the bent walls 10, 20. FIG. 6A showing a cross-sectional view that is perpendicular to an axis of longitudinal extension CA of the screw extender 100, that shows the arcuate or curved shape of walls 10, 20 with a bending radius, and FIG. 6B showing a cross-sectional view in a direction of the axis of longitudinal extension CA.

FIG. 7 shows another embodiment of the present invention, showing a proximal side of the screw extender 100 that is made of two separate parts by walls 10, 20, that can be interconnected with each other, by the use of two complementary cut sections or openings 464, 564 of the walls 10, 20. For example, each wall edge can include a tooth 468, 568 that can press-fittedly engage with a corresponding cavity or opening 464, 564. Openings 464, 564 or teeth 468, 568 can be formed by stamping, laser cutting, machining, for example before a step of bending the walls 10, 20 to their arcuate shape. This allows to create two shells with walls 10, 20 that can be connected to each other without the need of another attachment mechanism, for example without the need of threadable nut or other device for holding walls 10, 20 together.

Further, a method is provided that permits to manufacture a screw extender 100 without the need of performing any time-consuming and cost-ineffective CNC machining or molding that require a costly prefabricated mold, for example a mold for plastic or composite material injection to form a screw extender. More generally, an example is a method of manufacturing an internal thread into two pieces of flat metal plates or strips. For example, the method can include a step of providing a bar or strip of material 90, for example a metal strip, more specifically a strip of stainless steel, a step of cutting the strip of material 90 by a cutting process to form cuts 162, 164 into strip of material 90, for example by laser cutting or a cutting stamp. Wall-traversing cuts 162, 164 delineate or define tabs 168 that will form flanks of an internal thread, and are arranged as two sets of tabs 168 at two locations of the strip of material, respectively, arranged to be substantially axi-symmetrical to a middle axis MA (see FIG. 1A). Moreover, the method includes a step of cutting the strip of material 90 by a cutting process to form slits 264, 364 into strip of material 90 to form ridges for handle attachment mechanism 60 or ridges for the screw head attachment mechanism 55, or both. It is possible that the cutting steps are performed simultaneously, for example to cut the outline of the strip of material 90 as shown in FIG. 1A, and simultaneously cut the slits or cuts 162, 164, 264, 364, and opening 33 with a laser cutting or stamping step.

Moreover, the method can include two or more bending steps, for example a first step of bending that is performed after the cutting steps for bending the strip of material 90 into an arcuate or curved shape to have a bending radius R, so that walls 10, 20 substantially form sections of a cylindrical surface, for example by using a press brake with corresponding bending dies, and a step of bending or folding the strip of material 90 at two different locations L1 and L2 by 90° to form the U-shape, to define the walls 10, 20. Preferably, bending radius R is chosen to be slightly larger of a major radius of the external thread of device 105, that needs to threadably engage with threading structures 16, 26. The location L1 and L2 of the bending or folding in this step to form the two 90° bends for obtaining the U-shape requires a correct definition, to make sure that the pitches of threading structures 16, 26 are arranged at a defined geometric relationship to each other, first to make sure that a distance between walls 10, 20 that have been formed matches the major diameter of the internal thread of required threading structure 16, 26, and second to make sure that the offset of the thread pitches of the opposing threading structures 16, 26 are matching for threadable engagement of a device 105 that has the corresponding outer threading, for example a rod or cylinder. Before the step of bending the strip of material 90 into a U-shape, an optional bending step or steps can be performed to bend side edges of the strip of material 90 to form to form folds 80, for example four (4) folds 80, 180 that lie opposite to each other in pairs, to provide for additional stiffening of the screw extender 100, as shown in the cross-sectional views of FIGs. 2L and 3. The bending of strip of material 90 can also bend the curved or arcuate shape and the folds 80, 180 at the same time with dedicated die pairs of the press brake. This step can also include other bending operations, for example the widening the distal end for creating a widened space for accommodating a screw head and to create the correct width or diameter of the screw attachment mechanism 55, or the widening or narrowing the proximal end for creating a correct diameter for a handle attachment mechanism 60. After the bending of the curved or arcuate shape of walls 10, 20, and after the bending of the folds 80, 180, a separate bending step can be performed to bend tabs 168 inwards towards the opposing wall 10, 20, and a bending step to bend the tabs outwardly in a radial direction away from a center axis CA to form ridges 268, 368, for example by dedicated die pair as explained with FIGs. 6A and 6B, and also bending tabs inwardly to form ridges 52. Complementary dies of a die pair can be used for stamping the flared or widened sections of the screw extender 100, for example screw attachment mechanism.

An significant advantage in manufacturing a screw extender 100 from a strip of metal, for example a strip of stainless steel 90 that can be used for a surgical procedure, and the use of simple stamping, cutting and bending steps, provide for substantial advantages over the state of the art where screw extenders are mostly made from an injection mold, or machined from a piece of material. First, very little material is used as the strip of material 90 can be cut to the right size, and can be chosen to be relatively thin, for example down to 1 mm or even less, allowing to reduce the costs. Second, the material can be chosen to be easily recyclable, so that the envisaged one-time use of the screw extender 100 does not create unrecyclable waste. Third, the manufacturing can be relatively fast, as no time-consuming and costly CNC machining or other material removal techniques are necessary. In addition, in conjunction with the fact that thin material can be used, the overall diameter of the screw extender 100 can be reduced, down to 12 mm or less, requiring less space in the surgical incision.

FIG. 8 shows a cross-sectional side view along axis CA of a screw extender 100 with a threaded device 105 located between walls 10, 20, for example a rod reduction tool, set screw driver, screw driver, or screw release instrument, where bend tabs 168 are shown that form the threading structures 16, 26 for walls 10, 20, respectively, threaded device 105 having a corresponding external thread for engaging with threading structures 16, 26. Moreover, ridges 268, 368 are formed at the proximal side of screw extender 100, to form handle attachment mechanism 60. Ridges 268, 368 have been formed by bumping out or bending the tabs that form ridges 268, 368 in a radial direction away from center axis CA, in other words towards the convex side of the of cylindrical surfaces formed by walls 10, 20. Moreover, ridges 52 gave been formed that form screw head attachment mechanism 55, formed by bumping out or bending the tabs that form ridges 52 in a radial direction towards the center axis CA, in other words towards the concave side of cylindrical surfaces formed by walls 10, 20. In this variant, only two ridges 268, 368 or two ridges 52 are formed, but there can be more than two, for example four (4) or more, as exemplarily shown in FIG. 2C. The front or distal part of screw extender 100 also shows the flared or widened end to accommodate the width of a screw head (see also FIGs. 2A-2C). This widened end can also be formed by pressing or bending step, for example simultaneously when bending out ridges 268, 368, 52 or tabs 168.

## Claims

1. A screw extender for an implant system having a distal side for holding a screw and a proximal side, the screw extender comprising:
two side walls facing each other and connected to each other, the two side walls connected to each other at the proximal side by a connection element;
wherein inner sides of portions of the two side walls that face each other include a threading, **characterised in that**
the threading includes a plurality of flanks, each flank including a tab that is bent inwardly towards the other side wall and a wall-traversing opening in the corresponding side wall delineating the flank.

2. The screw extender of claim 1, wherein the wall-traversing opening forms a U-shape around the bent tab.

3. The screw extender of claim 1, wherein the distal side of each wall includes a first engagement mechanism for holding a pedicle screw, the first engagement mechanism formed by at least one protruding ridge and a slit-shaped wall-traversing opening on both sides of the ridge.

4. The screw extender of claim 1, wherein the proximal side of each wall includes a second engagement mechanism for holding a handle, the second engagement mechanism formed by at least one protruding ridge and a slit-shaped wall-traversing opening formed on both sides of the ridge.

5. The screw extender of claim 1, wherein the two side walls connected to each other at the proximal side by a connection element, the two side walls and the connection element are formed from one strip of material, the strip of material bent at two locations by ninety degrees, the connection element located between the two bent locations.

6. The screw extender of claim 1, wherein the two side walls and the connection element are separate elements that are connected to each other to form a U-shape.

7. The screw extender of claim 3, wherein the ridge of the first engagement mechanism is arranged to be parallel to an axis of longitudinal extension of the two side walls.

8. The screw extender of claim 1, wherein at least the inner sides of the portions of the two side walls with the threading are bent to form an arcuate shape when viewed in a direction of an axis of longitudinal extension of the two side walls.

9. The screw extender of claim 1, wherein at least one of the two side walls includes a bent fold along a long side of the corresponding side wall, the bent fold bent along a bending line that is parallel to a longitudinal axis of extension CA of screw extender, for stiffening the screw extender.

10. A method of manufacturing a screw extender for an orthopedic implant kit comprising the steps of:
cutting a strip of metal material to form two sets of wall-traversing openings, the sets of wall-traversing openings arranged to be axisymmetric to each other, the wall-traversing openings delineating tabs, the strip of metal material having a longitudinal axis of extension CA;
axially bending the strip of material around the longitudinal axis to have a curved cross-section with a bending radius;
folding the strip of s material at a first location to form a 90° angle and at a second location to form another 90° angle to provide for a U-shaped structure having two parallelly-arranged walls, the sets of wall-traversing openings arranged to face each other; and
inwardly bending the tabs towards an opposite wall to form flanks for an internal thread.

11. The method of manufacturing a screw extender according to claim 10, wherein wall-traversing openings include a slit, the slit being arranged at a threading angle that is oblique relative to an axis that is perpendicular to the longitudinal axis of extension CA.

12. The method of manufacturing a screw extender according to claim 10, further comprising a step of.
bending edges of the two parallelly-arranged walls to form folds.

13. The method of manufacturing a screw extender according to claim 10, further comprising a step of.
cutting the strip of metal material to form a central hole;

14. The method of manufacturing a screw extender according to claim 10, further comprising a step of.
cutting the strip of metal material at the two ends to form two parallelly arranged wall-traversing slits, the slits extending parallel to a direction of the longitudinal axis of extension CA.

15. The method of manufacturing a screw extender according to claim 14, further comprising a step of:
inwardly bending a tab towards an opposite wall, the tab being arranged between the two parallelly arranged wall-traversing slits, to form a ridge.

## Patentansprüche

1. Schrauben-Extender für ein Implantatsystem mit einer distalen Seite zum Halten einer Schraube und einer proximalen Seite, wobei der Schrauben-Extender Folgendes umfasst:
zwei Seitenwände, die einander zugewandt und miteinander verbunden sind, wobei die beiden Seitenwände über ein Verbindungselement an der proximalen Seite miteinander verbunden sind,
wobei innere Seiten von Abschnitten der beiden Seitenwände, die einander zugewandt sind, ein Gewinde aufweisen, **dadurch gekennzeichnet, dass**
das Gewinde eine Vielzahl von Flanken aufweist, wobei jede Flanke eine Lasche aufweist, die nach innen auf die andere Seitenwand zu gebogen ist, sowie eine durch die Wand gehende Öffnung in der entsprechenden Seitenwand, die die Flanke abgrenzt.

2. Schrauben-Extender nach Anspruch 1, wobei die durch die Wand gehende Öffnung eine U-Form um die gebogene Lasche bildet.

3. Schrauben-Extender nach Anspruch 1, wobei die distale Seite jeder Wand einen ersten Eingriffsmechanismus zum Halten einer Pedikelschraube aufweist, wobei der erste Eingriffsmechanismus von mindestens einer vorragenden Erhöhung und einer schlitzförmigen, durch die Wand gehende Öffnung an beiden Seiten der Erhöhung gebildet wird.

4. Schrauben-Extender nach Anspruch 1, wobei die proximale Seite jeder Wand einen zweiten Eingriffsmechanismus zum Halten eines Griffs aufweist, wobei der zweite Eingriffsmechanismus von mindestens einer vorragenden Erhöhung und einer schlitzförmigen, durch die Wand gehende Öffnung an beiden Seiten der Erhöhung gebildet wird.

5. Schrauben-Extender nach Anspruch 1, wobei die beiden Seitenwände über ein Verbindungselement an der proximalen Seiten miteinander verbunden sind, wobei die beiden Seitenwände und das Verbindungselement aus einem Materialstreifen ausgebildet sind, wobei der Materialstreifen an zwei Stellen um neunzig Grad gebogen ist und das Verbindungselement zwischen den beiden gebogenen Stellen angeordnet ist.

6. Schrauben-Extender nach Anspruch 1, wobei die beiden Seitenwände und das Verbindungselement separate Elemente sind, die unter Bildung einer U-Form miteinander verbunden sind.

7. Schrauben-Extender nach Anspruch 3, wobei die Erhöhung des ersten Eingriffsmechanismus so angeordnet ist, dass sie parallel zu einer Längserstreckungsachse der beiden Seitenwände verläuft.

8. Schrauben-Extender nach Anspruch 1, wobei mindestens die inneren Seiten der Abschnitte der beiden Seitenwände mit dem Gewinde so gebogen sind, dass sie mit Blick in einer Richtung einer Längserstreckungsachse der beiden Seitenwände eine bogenförmige Gestalt bilden.

9. Schrauben-Extender nach Anspruch 1, wobei mindestens eine der beiden Seitenwände eine gebogene Faltung entlang einer langen Seite der entsprechenden Seitenwand aufweist, wobei die gebogene Faltung entlang einer Biegelinie gebogen ist, die parallel zu einer Längserstreckungsachse CA des Schrauben-Extenders verläuft, um den Schrauben-Extender zu versteifen.

10. Verfahren zur Herstellung eines Schrauben-Extenders für einen orthopädischen Implantatsatz, umfassend die folgenden Schritte:
Schneiden eines Metallmaterialstreifens zum Bilden von zwei Sätzen von durch die Wand gehenden Öffnungen, wobei die Sätze von durch die Wand gehenden Öffnungen achsensymmetrisch zueinander angeordnet sind, wobei die durch die Wand gehenden Öffnungen Laschen abgrenzen, wobei der Metallmaterialstreifen eine Längserstreckungsachse CA hat,
axiales Biegen des Materialstreifens um die Längsachse zum Erhalten eines gekrümmten Querschnitts mit einem Biegeradius,
Falten des Materialstreifens an einer ersten Stelle zum Bilden eines 90°-Winkels und an einer zweiten Stelle zum Bilden eines weiteren 90°-Winkels, um eine U-förmige Struktur mit zwei parallel angeordneten Wänden bereitzustellen, wobei die Sätze von durch die Wand gehenden Öffnungen so angeordnet sind, dass sie einander zugewandt sind,
und
Biegen der Laschen nach innen zu einer gegenüberliegenden Wand hin, um Flanken für ein Innengewinde zu bilden.

11. Verfahren zur Herstellung eines Schrauben-Extenders nach Anspruch 10, wobei durch die Wand gehende Öffnungen einen Schlitz aufweisen, wobei der Schlitz mit einem Flankenwinkel angeordnet ist, der bezüglich einer senkrecht zu der Längserstreckungsachse CA verlaufenden Achse schräg ist.

12. Verfahren zur Herstellung eines Schrauben-Extenders nach Anspruch 10, ferner umfassend einen Schritt:
des Biegens von Rändern der beiden parallel angeordneten Wände zum Bilden von Falten.

13. Verfahren zur Herstellung eines Schrauben-Extenders nach Anspruch 10, ferner umfassend einen Schritt:
des Schneidens des Metallmaterialstreifens zum Bilden eines mittleren Lochs.

14. Verfahren zur Herstellung eines Schrauben-Extenders nach Anspruch 10, ferner umfassend einen Schritt:
des Schneidens des Metallmaterialstreifens an den beiden Enden zum Bilden von zwei parallel angeordneten durch die Wand gehenden Schlitzen, wobei sich die Schlitze parallel zu einer Richtung der Längserstreckungsachse CA erstrecken.

15. Verfahren zur Herstellung eines Schrauben-Extenders nach Anspruch 14, ferner umfassend einen Schritt:
des Biegens einer Lasche nach innen zu einer gegenüberliegenden Wand hin, wobei die Lasche zwischen den beiden parallel angeordneten, durch die Wand gehenden Schlitzen angeordnet ist, um eine Erhöhung zu bilden.

## Revendications

1. Prolongateur de vis pour un système d'implant ayant un côté distal pour tenir une vis et un côté proximal, le prolongateur de vis comprenant :
deux parois latérales se faisant face et reliées l'une à l'autre, les deux parois latérales étant reliées l'une à l'autre au niveau du côté proximal par un élément de liaison ;
des côtés intérieurs des parties des deux parois latérales qui se font face comprenant un filetage, **caractérisé en ce que** le filetage comprend plusieurs flancs, chaque flanc comprenant une languette qui est courbée vers l'intérieur en direction de l'autre paroi latérale et une ouverture traversant la paroi dans la paroi latérale correspondante délimitant le flanc.

2. Prolongateur de vis selon la revendication 1, l'ouverture traversant la paroi formant une forme de U autour de la languette courbée.

3. Prolongateur de vis selon la revendication 1, le côté distal de chaque paroi comprenant un premier mécanisme de mise en prise pour maintenir une vis pédiculaire, le premier mécanisme de mise en prise étant formé par au moins une arête saillante et une ouverture de traversée de paroi en forme de fente de part et d'autre de l'arête.

4. Prolongateur de vis selon la revendication 1, le côté proximal de chaque paroi comprenant un second mécanisme de mise en prise pour tenir une poignée, le second mécanisme de mise en prise étant formé par au moins une arête saillante et une ouverture traversant la paroi en forme de fente formée de part et d'autre de l'arête.

5. Prolongateur de vis selon la revendication 1, les deux parois latérales étant reliées l'une à l'autre du côté proximal par un élément de liaison, les deux parois latérales et l'élément de liaison étant formés d'une bande de matériau, la bande de matériau étant courbée à deux endroits à quatre-vingt-dix degrés, l'élément de liaison étant situé entre les deux endroits courbés.

6. Prolongateur de vis selon la revendication 1, les deux parois latérales et l'élément de liaison étant des éléments séparés qui sont reliés l'un à l'autre pour former une forme de U.

7. Prolongateur de vis selon la revendication 3, l'arête du premier mécanisme de mise en prise étant agencée de manière à être parallèle à un axe d'extension longitudinale des deux parois latérales.

8. Prolongateur de vis selon la revendication 1, au moins les côtés intérieurs des parties des deux parois latérales avec le filetage étant courbés pour former une forme arquée lorsqu'ils sont vus dans la direction d'un axe d'extension longitudinale des deux parois latérales.

9. Prolongateur de vis selon la revendication 1, au moins l'une des deux parois latérales comprenant un pli courbé le long d'un côté long de la paroi latérale correspondante, le pli courbé le long d'une ligne de courbure qui est parallèle à un axe longitudinal d'extension CA du prolongateur de vis, pour raidir le prolongateur de vis.

10. Procédé de fabrication d'un prolongateur de vis pour un nécessaire d'implant orthopédique comprenant les étapes de :
découpage d'une bande de matériau métallique pour former deux ensembles d'ouvertures traversant la paroi, les ensembles d'ouvertures traversant la paroi étant agencés de manière à être axisymétriques l'un par rapport à l'autre, les ouvertures traversant la paroi délimitant des languettes, la bande de matériau métallique ayant un axe longitudinal d'extension CA ;
courbure axialement de la bande de matériau autour de l'axe longitudinal pour obtenir une section transversale incurvée avec un rayon de courbure ;
courbure de la bande de matériau à un premier endroit pour former un angle de 90° et à un deuxième endroit pour former un autre angle de 90° afin d'obtenir une structure en forme de U ayant deux parois agencées parallèlement, les ensembles d'ouvertures traversant les parois étant agencés l'un en face de l'autre ; et
courbure vers l'intérieur des languettes vers une paroi opposée pour former des flancs pour un filetage interne.

11. Procédé de fabrication d'un prolongateur de vis selon la revendication 10, les ouvertures traversant les parois comprenant une fente, la fente étant agencée selon un angle de filetage oblique par rapport à un axe perpendiculaire à l'axe longitudinal du prolongateur CA.

12. Procédé de fabrication d'un prolongateur de vis selon la revendication 10, comprenant en outre une étape de :
courbure des bords des deux parois agencées parallèlement pour former des plis.

13. Procédé de fabrication d'un prolongateur de vis selon la revendication 10, comprenant en outre une étape de :
découpage de la bande de matériau métallique pour former un trou central ;

14. Procédé de fabrication d'un prolongateur de vis selon la revendication 10, comprenant en outre une étape de :
découpage de la bande de matériau métallique aux deux extrémités pour former deux fentes traversant la paroi et agencées parallèlement, les fentes s'étendant parallèlement à une direction de l'axe longitudinal de l'extension CA.

15. Procédé de fabrication d'un prolongateur de vis selon la revendication 14, comprenant en outre une étape de :
courbure vers l'intérieur d'une languette vers une paroi opposée, la languette étant agencée entre les deux fentes agencées en parallèle traversant la paroi, pour former une arête.
